# EUROPEAN PATENT APPLICATION

(11) **EP 1 532 942 A1**
(43) Date of publication of application: **25.05.2005**
(21) Application number: 02807600.8
(22) Date of filing: 10.07.2002
(51) Int. Cl.: A61F 2/02

(54) **ENDOPROSTHESIS FOR REPARATIVE ANAPLASTIC SURGERY**

(71) Applicant: Sulamanidze, Marlen Andreevich, Moscow, 115533 (RU); Sulamanidze, Georgiih Marlenovich, Moscow, 115533 (RU)
(72) Inventor: Sulamanidze, Marlen Andreevich, Moscow, 115533 (RU); Sulamanidze, Georgiih Marlenovich, Moscow, 115533 (RU)
(74) Representative: Hano, Christian, Dipl.-Ing.
(86) International application number: PCT/RU2002/000331
(87) International publication number: WO 2004/006806

(57) **Abstract**

The inventive strip-shaped endoprosthesis (1) for reparative anaplastic surgery comprises a cover (2) whose surface (3) is provided with protrusions (4) which are embodied in the form of inclined conical barbs (6) having flexible and elastic sharpened ends (5). The protruded barbs are differently disposed on the endoprosthesis surface in order to improve the fixation thereof to soft tissues. The embodiments of said endoprosthesis are selected according to the purpose thereof. The embodiments of volume endoprosthesises (9 and 14) for eliminating the breast ptosis and correcting a dorsum of nose are also enclosed. The protruded barbs are applied on the cover in such a way than the resistance of the endoprosthesis is preserved.

## Description

### Technical field

The invention relates to medicine and to methods for carrying out surgical and plastic operations. In particular, it relates to endoprostheses used in reparative anaplastic and aesthetic operations.

### Background art

Endoprostheses for anaplastic and aesthetic surgery are known (Ya. Zoltan, Reconstruction of a feminine mammary gland, Hungary Academy of Sciences, 1989. N. M. Aleksandrov, Clinical operative maxillofacial surgery, Medicine, Moscow, 1985, p.94-95).

The known endoprostheses are made from different polymeric and biological materials and comprise covers, the general characteristics of which are strength, a cellular, smooth, homogeneous or textural surface, density, bioinertia.

After implantation in a region of a defect endoprostheses must substitute the defect, eliminate an outline deformation and preserve the postoperative result for a long time. The known cellular endoprostheses are usually accurately sewed to the edge of a defect and only after that they begin to perform a supporting function.

The cellular endoprostheses are also used in plastic operations of mammary glands, for example mastopexy. For strengthening and fixing the mammary glands nets are intraoperatively sewed around the mammary glands.

Endoprostheses with a textural surface are also used, for example prostheses of the firm McGan for increasing mammoplasty.

Connective tissue tightening on the surface of such prostheses cannot provide further reliable fixation. Eventually the gravity makes the prostheses slide down and the mammary glands hang down, and deteriorates the aesthetic results.

The same deterioration of postoperative aesthetic results takes place in rinoplastics when in the course of time the tip of the nose slides off a silicone endoprosthesis and hangs over the lip.

The closest to the claimed invention is an endoprosthesis, the cover of which is provided with a textural surface. The Textural surface comprises roughness between the threads penetrated by the connective tissue. But this is not enough for stable and long keeping of the endoprosthesis in soft tissues.

We propose a new surface of endoprostheses which allows to fix not only an implantant itself without usual sewing thereof to soft tissues but also soft tissues themselves.

### The disclosure of the invention

The raised problem is solved in that an endoprosthesis for anaplastic and aesthetic surgery made from polymeric and bionert material, comprises a cover, a surface of which is provided with protrusions in the form of inclined conical flexible elastic barbs having sharpened ends, the direction, the arrangement, and also the interval between the barbs being made different in dependence of the functional purpose of the endoprosthesis and the elevation of the ends of the protrusions above the surface being from 0.01 to 5 mm also corresponding to the purpose.

The problem underlying the invention is to design a surgical endoprosthesis, the cover of which is firmly fixed on an organism and prevents both prosthesis and soft tissues from moving caused by body movement and also by gravity influences on the organism.

The raised problem is solved by the use of the above mentioned cover of the endoprosthesis.

It is also advisable that the protrusions-barbs of the strip-shaped Oendoprosthesis are provided on one or two sides of the strip in one direction and arranged in staggered order.

It is also advisable to provide the protrusions-barbs on two sides directed oppositely from the center of the belt-formed endoprosthesis.

It is also possible to alternate the directions of the protrusions-barbs every one row.

Endoprostheses of three-dimensional form can have various variants of arrangement of protrusions -barbs:
- barbs directed in one direction on one side
- it is also possible to alternate the directions of the inclination of the protrusions-barbs on each of the sides.

### Brief description of the figures

The proposed invention is explained by specific examples of its realization and by drawings showing the following:
Fig. 1- shows a variant of a strip-shaped endoprosthesis with protrusions-barbs on one side.
Fig.2 - shows the side view of figure 1.
Fig.3 - shows a variant of a strip-shaped endoprosthesis with protrusions-barbs on two sides.
Fig.4 - shows the side view of figure 3.
Fig.5 - shows a variant of a strip-shaped endoprosthesis with protrusions-barbs on two sides directed oppositely from its center.
Fig.6 - shows the side view of figure 5.
Fig.7 - shows a variant of a strip-shaped endoprosthesis with the every one row alternation of protrusions-barbs on its two sides.
Fig.8- shows the side view of figure 7.
Fig.9 - shows an endoprosthesis of three-dimensional form with protrusions-barbs arranged on one side in one directions along its surface.
Fig. 10 - shows an endoprosthesis of three-dimensional form with alternating inclination of the protrusions-barbs provided on each of the sides.
Fig.11 - shows schematically a surgical operation for correction of the bridge of the nose.
Fig.12 - shows a surgical operation for correction of the bridge of the nose with a variant of endoprosthesis with the protrusions-barbs directed differently along its surface.

### Basic variant of carrying out the invention

An endoprosthesis 1 for reparative anaplastic surgery is strip-shaped and has a cover 2, a surface 3 of which is provided with protrusions 4 in the form of inclined conical flexible elastic barbs 6 with sharpened ends 5. The interval 7 between the barbs 6 varies according to the functional purpose of the endoprosthesis. The elevation 8 of the ends 5 of the protrusions 5 above the surface can also vary from 0.01 to 5 mm according to the purpose of the endoprosthesis.

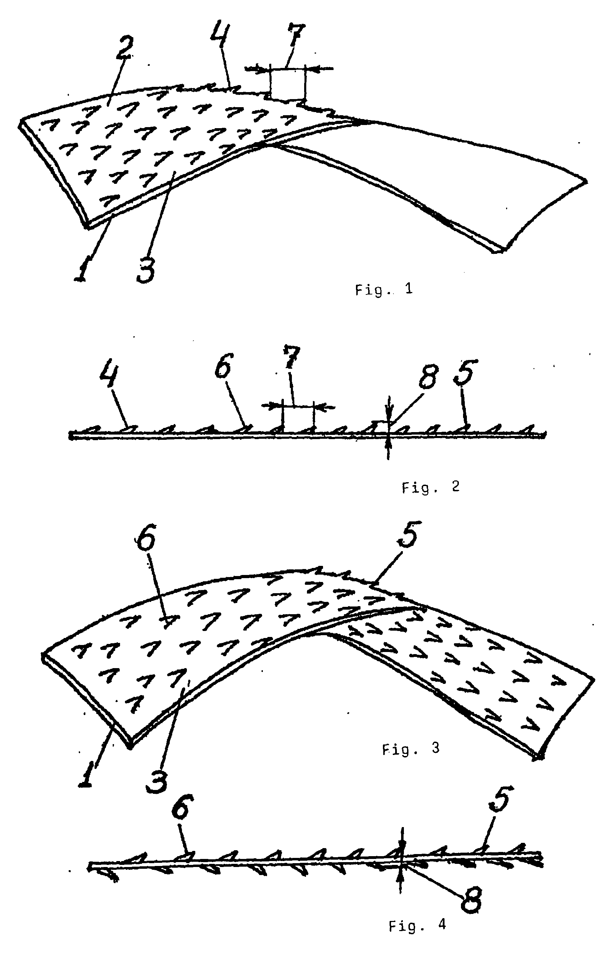

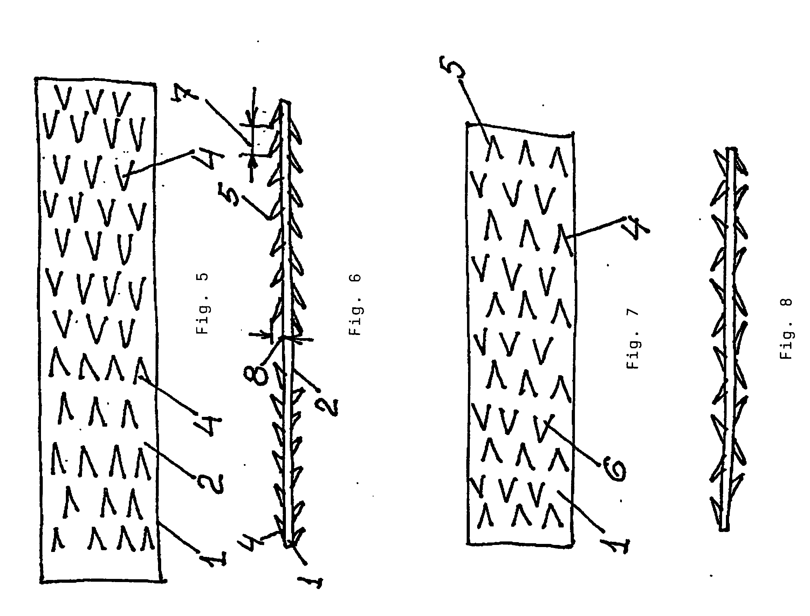

Fig.1, 2, 3, 4 shown the various arrangements of protrusions-barbs on the surface of the cover of the endoprosthesis: directed in one direction on one and two sides of the strip-shaped endoprosthesis, directed oppositely from the center of the strip-shaped endoprosthesis.(fig.5 and 6)

Fig.7 and 8 show a strip-shaped endoprosthesis, the direction of the protrusions-barbs on its two sides alternating every one row. It is preferable to arrange the protrusions in staggered order to preserve and strengthen the cover of the endoprosthesis and to provide a better fixation thereof to soft tissues.

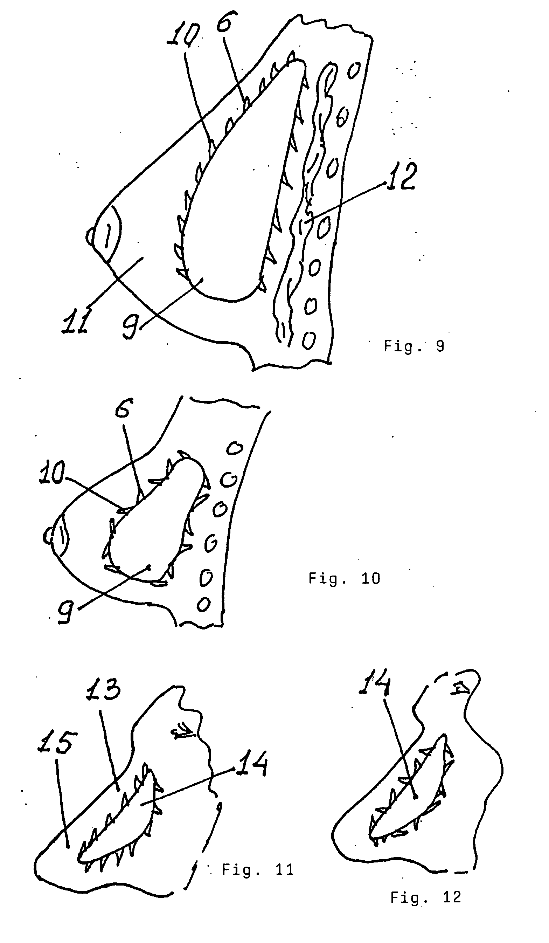

Fig.9 shows a three-dimensional endoprosthesis 9 on the surface of which protrusions-barbs 6 are formed in one direction on one side of the cover 10.

Fig.10 shows a three-dimensional endoprosthesis having alternating inclinations of the protrusions-barbs 6 provided on each of the sides.

The effectiveness of the use of the endoprosthesis is shown in fig.12 illustrating schematically a variant of the use of the three-dimensional endoprosthesis 9 with protrusions-barbs 6 for removal of ptosis of a feminine mammary gland 11. Position 12 corresponds to a muscle.

Surgical operation for removal of sagging of soft tissues of a neck is made in the following way. Skin adipose tissue is cut by means of a wire scalpel along a preliminary marked contour, thus making a hypodermic tunnel in the area of neck, then in parotid areas from both sides 1,5 - 2 cm skin is cut. Through these cuts along the above mentioned tunnel cellular strip-shaped endoprosthesis is stretched, then with the required force it is pulled from both sides and pressed to the soft tissues. At the same time the protrusions-barbs 6 on the strip cling on the wound surface from the side of the skin and the side of the platisma for fixing the strip in the necessary position, thereby removing the sagging of the neck. After that the wound is sewed. Fig.11 shows schematically a surgical operation for correction of the bridge of a nose 13, the endoprosthesis 14, the skin and hypodermic cellulose 15.

### Industrial applicability

As it is shown the invention can be widely used in different reparative anaplastic plastic and aesthetic surgery operations.

### References

1. Ya. Zoltan, <Reconstruction of a feminine mammary gland>, Hungary Academy of Sciences, 19 89. (prototype).
2. N. M. Aleksandrov, <Clinical operative maxillofacial surgery>, <Medicine>, Moscow, 1985, p.94-95).

## Claims

1. Endoprosthesis (1) for anaplastic and aesthetic surgery made from polymeric biological material comprising the cover (2) with a textured, cellular or rough surface, **characterized in that** the surface (3) of the cover (2) is provided with protrusions (4) in the form of inclined conical flexible elastic barbs (6) with sharpened ends (5), wherein the direction, the arrangement and the interval (7) between the barbs (6) vary in dependence of the functional purpose of the endoprosthesis, and the elevation (8) of the ends of the protrusions above the surface is from 0.01 mm to 5 mm also according to the purpose of the endoprosthesis.

2. Endoprosthesis (1) according to claim 1 **characterized in that** the protrusion-barbs (6) provided on one side have the same direction and are arranged in a staggered order.

3. Endoprosthesis (1) according to claim 1 and 2 **characterized in that** the protrusion-barbs (6) are provided on both sides of the endoprosthesis.

4. Endoprosthesis (1) according to claim 1 and 2 **characterized in that** the protrusions-barbs (6) are provided on both sides of the endoprosthesis and are directed opposite each other from the center thereof.

5. Endoprosthesis (1) according to claim 1 and 2 **characterized in that** the protrusions-barbs (6) are provided on two sides of the endoprosthesis with every row alternation of their directions.

6. Endoprosthesis (1) according to claim 1 and 2 **characterized in that** it is made of three-dimensional form and is provided on one side with the barbs (6) having the same direction along the whole surface.

7. Endoprosthesis (1) according to claim 1 and 2 **characterized in that** it is made of three-dimensional form alternating inclination directions of the protrusions-barbs (6) on each of the sides.
